# EUROPÄISCHE PATENTSCHRIFT

(11) **EP 1 737 817 B1**
(45) Veröffentlichungstag und Bekanntmachung des Hinweises auf die Patenterteilung: **02.12.2009**
(21) Anmeldenummer: 04819649.7
(22) Anmeldetag: 02.12.2004
(51) Int. Cl.: C07D 205/06, C08J 3/24, C08F 8/32, C08K 5/3412

(54) **AZETIDIN-DERIVATE, VERFAHREN ZU IHRER HERSTELLUNG SOWIE DEREN VERWENDUNG**
AZETIDINE DERIVATIVES, METHOD FOR PRODUCING SAID DERIVATIVES AND USE THEREOF
DERIVES D'AZETIDINE, LEURS PROCEDES DE PRODUCTION ET LEUR UTILISATION

(30) Priorität: 03.12.2003 DE 10356489
(43) Veröffentlichungstag der Anmeldung: 03.01.2007
(73) Patentinhaber: Construction Research & Technology GmbH, 83308 Trostberg (DE)
(72) Erfinder: INGRISCH, Stefan, 83358 Seebruck (DE); MAIER, Alois, 84549 Engelsberg (DE); PFEUFFER, Thomas, 83308 Trostberg (DE); STEIDL, Norbert, 83361 Kienberg (DE); WINKELMANN, Herbert, 84518 Garching (DE)
(74) Vertreter: Dey, Michael
(86) Internationale Anmeldenummer: PCT/EP2004/013730
(87) Internationale Veröffentlichungsnummer: WO 2005/054185

(56) Entgegenhaltungen:
- GB-A- 858 038
- US-A- 4 576 980
- US-A- 4 880 869
- US-A- 5 276 166
- DATABASE CAPLUS [Online] CHEMICAL ABSTRACTS SERVICE, COLUMBUS, OHIO, US; MARINETTI, ANGELA ET AL: "Enantioselective preparation of 2,4-disubstituted azetidines" XP002319678 gefunden im STN Database accession no. 2000:336170 & EUROPEAN JOURNAL OF ORGANIC CHEMISTRY , (9), 1815-1820 CODEN: EJOCFK; ISSN: 1434-193X, 2000,
- DATABASE CAPLUS [Online] CHEMICAL ABSTRACTS SERVICE, COLUMBUS, OHIO, US; TESTA, I. EMILIO ET AL: "Substances acting on the central nervous system. XIV. 3,3-Disubstituted azetidines" XP002319679 gefunden im STN Database accession no. 1962:45914 & ANN. , 633, 56-66, 1960,
- DATABASE CAPLUS [Online] CHEMICAL ABSTRACTS SERVICE, COLUMBUS, OHIO, US; MURAKAMI, MASUO ET AL: "Diazoniadispiroalkane salts" XP002319680 gefunden im STN Database accession no. 1970:111531 & JP 450 027 51B B4 (YAMANOUCHI PHARMACEUTICAL CO., LTD.) 29. Januar 1970 (1970-01-29)
- DATABASE CAPLUS [Online] CHEMICAL ABSTRACTS SERVICE, COLUMBUS, OHIO, US; LUNAK, STANISLAV ET AL: "Hardenable epoxy compositions with increased storage stability" XP002319681 gefunden im STN Database accession no. 1986:554121 & CS 224 495 B (CZECH.) 16. Januar 1984 (1984-01-16)

## Beschreibung

Die vorliegende Erfindung betrifft Azetidin-Derivate, Verfahren zu ihrer Herstellung sowie deren Verwendung als latente Härterkomponente für Harze, die gegenüber Aminen reaktive funktionelle Gruppen aufweisen, insbesondere Polyurethan- und Polyepoxid-Harze.

Latente Härterkomponenten werden insbesondere für feuchtigkeitserhärtende Polyurethanmassen bei der Herstellung von Dichtmassen, Klebstoffen und Beschichtungsmitteln eingesetzt. Im Stand der Technik sind schon eine ganze Reihe von latenten Härtern beschrieben worden, die jedoch alle den gravierenden Nachteil aufweisen, dass während der Härtungsreaktion leichtflüchtige organische Verbindungen freigesetzt werden, welche entweder die Umwelt belasten und/oder gesundheitlich problematisch erscheinen.

So werden gemäß der DE-OS 30 19 356 als Härter für Polyisocyanate Aldimin- und Oxazolidingruppen aufweisende Verbindungen beschrieben, die a) durch Umsetzung von Polyaminen mit einer Epoxidverbindung und b) der anschließenden Cyclisierung der in Stufe a) gebildeten Polyaminoalkohole mit Aldehyden hergestellt werden. Bei der Aushärtung dieser Aldiminooxazolidine mit Polyisocyanaten werden in Gegenwart von Wasser oder atmosphärischer Feuchtigkeit Aldehyde abgespalten, die unter Umständen eine starke Geruchsbelästigung darstellen und deshalb nur im Außenbereich eingesetzt werden können.

Aus der DE-OS 36 24 924 sind feuchtigkeitshärtende, lagerstabile Einkomponenten-Polyurethansysteme bekannt, die neben dem Polyurethan-Prepolymer als erfindungswesentliche Komponente ein Polyaldimin als Härter enthalten. Auch bei diesen Polyurethansystemen werden während der Härtung Aldehyde abgespalten, wodurch eine Anwendung für den Innenbereich von vorneherein ausgeschlossen ist. Ein weiterer Nachteil bei diesen Polyurethan-Systemen ist die Tatsache, dass die entsprechenden Polyurethan-Prepolymere eine relativ hohe Viskosität aufweisen, so dass zur Verminderung der Viskosität Malonsäurediethylester zugesetzt werden müssen.

Entsprechend der DE-OS 40 21 659 werden Bisoxazolane als Härter für Polyurethansysteme empfohlen, die durch Umsetzung von Diethanolamin mit Aldehyden hergestellt werden. Zwar können auf diese Weise niedrigviskose und lösemittelfreie Produkte zur Verfügung gestellt werden, doch spalten auch diese Bisoxazolane während der Härtungsreaktion pro Mol Härter zwei Mol Aldehyd ab, was mit den bereits vorstehend beschriebenen Nachteilen verbunden ist.

Des Weiteren werden in der EP-A 291 850 Polyurethan-Einkomponenten-Systeme beschrieben, die neben dem Polyurethan-Prepolymer einen latenten Härter aus der Gruppe Oxazolidine, Enamine und Azomethine, vorzugsweise Ketimine und/oder Aldimine enthalten. Auch diese Verbindungen spalten bei der Hydrolyse in Gegenwart von Feuchtigkeit unerwünschte Aldehyde oder Ketone ab. Außerdem müssen den Polyurethan-Prepolymeren bzw. Polyurethan-Einkomponenten-Systemen zur Verminderung des Viskositätsanstiegs Malonsäurediethylester in einer Menge von bis zu 10 Gew.-% zugesetzt werden.

Gemäß der WO 95/11 933 werden Aldimin-Oxazolidine offenbart. Neben der relativ aufwendigen Herstellung muss die Freisetzung von Aldehyden bei der Härtungsreaktion dieser Verbindungen als besonders nachteilig angesehen werden.

Schließlich sind aus der EP-A 947 529 Polyurethan-Prepolymere bekannt, die neben den Isocyanat-Gruppen noch latente Aminogruppen aufweisen. Die Herstellung dieser Polyurethan-Prepolymere erfolgt durch Addition eines Amino-Aldimins oder eines Cycloaminals an die Isocyanatgruppe eines Polyurethan-Polymers. Auch bei diesem Polyurethan-System lässt sich die Abspaltung von Benzaldehyd während der Härtungsreaktion mit Hilfe von Wasser oder Luftfeuchtigkeit nicht vermeiden.

GB 858 038 A beschreibt gesättigte Acetidine. US-A-4,880,869 offenbart Lactame und US-A-5,276,166 Acetidinole.

US-A-4,576,980 beschreibt Azetidinedione. Darin wird die Verwendung dieser Azetidinedione als Härtekomponenten offenbart.

Der vorliegenden Erfindung lag daher die Aufgabe zugrunde, eine latente Härterkomponente für Harze mit für Amine reaktiven funktionellen Gruppen bereitzustellen, welche die genannten Nachteile des Standes der Technik nicht aufweist, sondern während der feuchtigkeitsinduzierten Härtung keine leichtflüchtigen organischen Verbindungen abspaltet, gute anwendungstechnische Eigenschaften besitzt und relativ einfach und kostengünstig hergestellt werden kann.

Diese Aufgabe wurde erfindungsgemäß durch Azetidin-Derivate der allgemeinen Formel (I) gemäß Anspruch 1 gelöst.

Es hat sich überraschenderweise gezeigt, dass mit der erfindungsgemäß bereitgestellten Härterkomponente während der Härtungsreaktion überhaupt keine organischen Verbindungen abgespaltet werden. Außerdem lassen sich die erfindungsgemäßen latenten Härter gut mit allen gängigen Isocyanat- bzw. Epoxid-funktionellen Systemen formulieren, wobei die entsprechenden Härter/Harz-Umsetzungsprodukte eine sehr gute Lagerstabilität über einen längeren Zeitraum aufweisen, was ebenfalls nicht vorhersehbar war.

Erfindungsgemäß wird als latente Härterkomponente ein Azetidin -Derivat der allgemeinen Formel (II) oder III bereitgestellt, 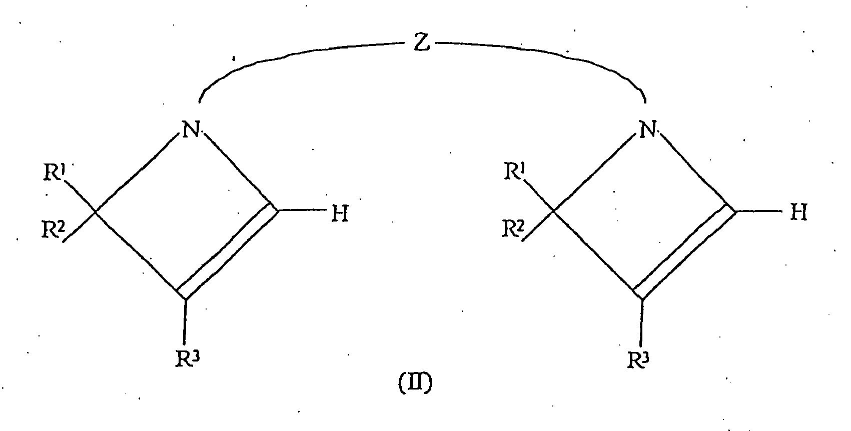 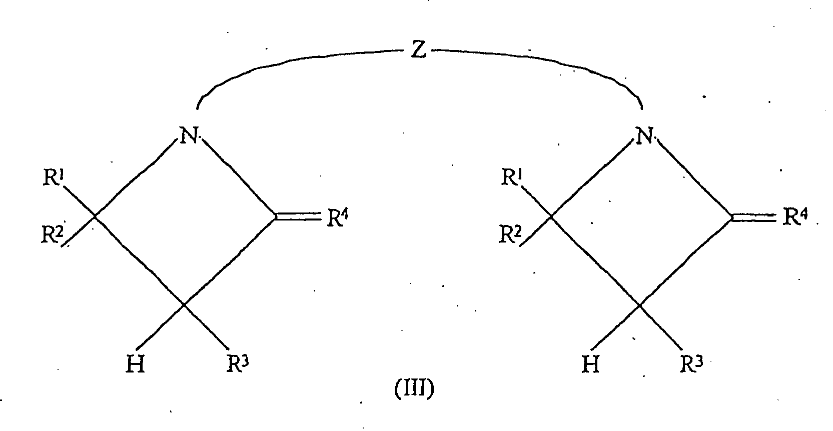 wobei
- R¹, R² und R³: unabhängig voneinander H, C₁-C₂₀-Alkyl, C₃-C₈-Cycloalkl, C₆-C₁₀-Aryl oder Alkylaryl mit C₁-C₄-Alkyl und C₆-C₁₀-Arylgruppen
- R⁴ =: C₁-C₆-Alkyl(iden)
- Z =: C₂-C₂₅-Alkylen, C₅-C₂₅-Cycloalkylen, C₆-C₂₄-Arylen sowie 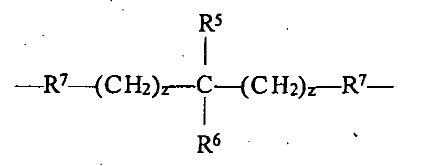
- R⁵ und R⁶ =: H, CH₂OH, C₁-C₄-Alkyl, C₆H₅, 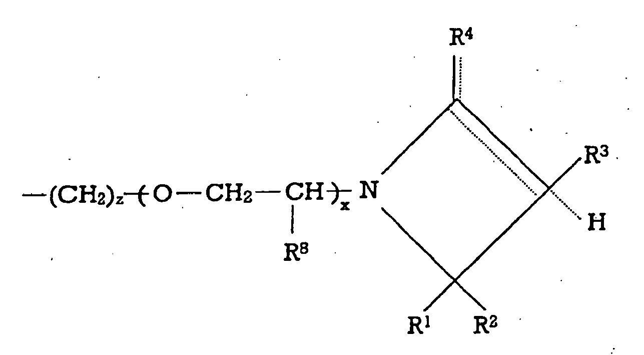
- R⁷: 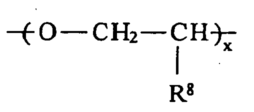
- R⁸ =: H, CH₃, C₂H₅, C₆H₅
- z =: 0 oder 1
- x =: 0 bis 100
bedeuten.

Bevorzugte Alkylreste, die entweder linear oder verzweigt sein können, sind C₁ bis C₄-Alkylgruppen. Bei den Cycloalkylresten sind Cyclopentyl- und Cyclohexylgruppen und bei den Arylresten Phenyl- und Naphthylgruppen als bevorzugt anzusehen.

Gemäß einer bevorzugten Ausführungsform werden vor allem Azetidin-Derivate der allgemeinen Formel (II) eingesetzt, 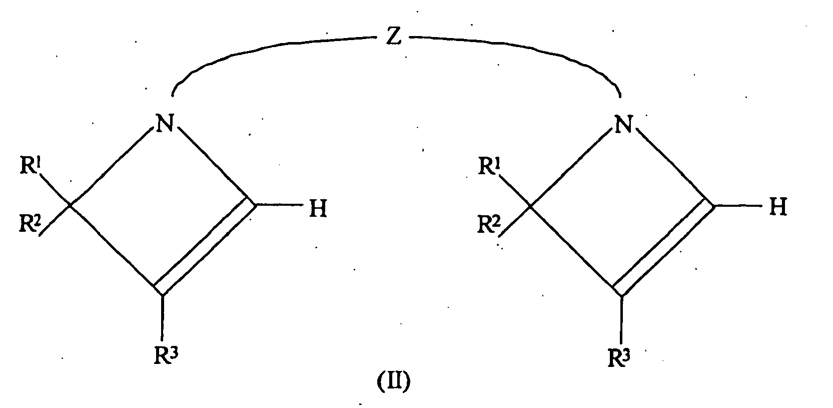
wobei R¹, R², R³ und Z oben genannte Bedeutung besitzen. Die Azetidin-Derivate gemäß der Formel (II) sind sehr einfach durch Umsetzung von einem Mol des Polyamins der Formel H₂N-Z'- NH₂ mit zwei Mol eines α,β-ungesättigten Aldehyds der Formel R¹R²-C=CR³CHO entsprechend der Gleichung (A) herstellbar, wobei unter Wasserabspaltung die Cyclisierung zum Bis-Azetidin-Derivat erfolgt: 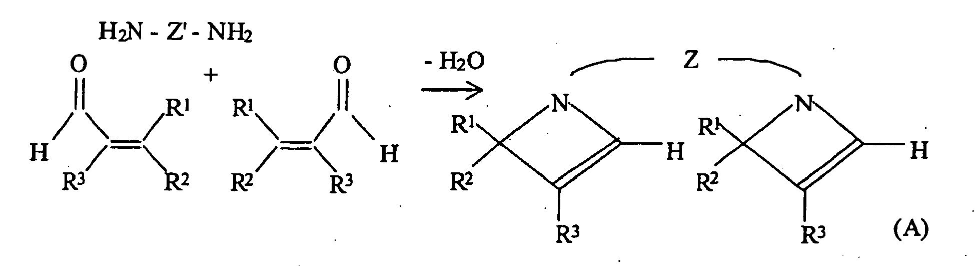
Z' hat hierbei folgende Bedeutung:
   C₂-C₂₅-Alkylen, C₅-C₂₅-Cycloalkylen, C₆-C₂₄-Arylen sowie 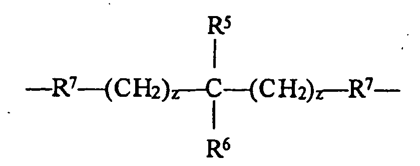
   R⁵ und R⁶ = H, CH₂OH, C₁-C₄-Alkyl, C₆H₅, 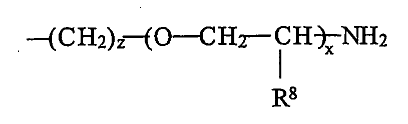 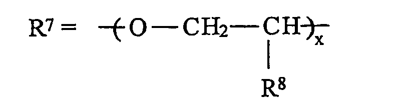
   R⁸ = H, CH₃, C₂H₅, C₆H₅
   z = 0 oder 1
   x = 0 bis 100

Für den Fall, dass das Polyamin NH₂-Z'-NH₂ drei oder vier NH₂-Gruppen aufweist, können durch Umsetzung mit drei oder vier Mol des α, β-ungesättigten Aldehyds die entsprechenden Tris- oder Tetrakis-Azetidine hergestellt werden.

Bei der Umsetzung eines Mols des Polyamins H₂N-Z'-NH₂ mit zwei Mol eines α, βungesättigten Ketons der Formel R¹R²C=C-CR³-COR⁴ entsprechend der Gleichung (B) entstehen Bis-Azetidin-Derivate der allgemeinen Formel (III):

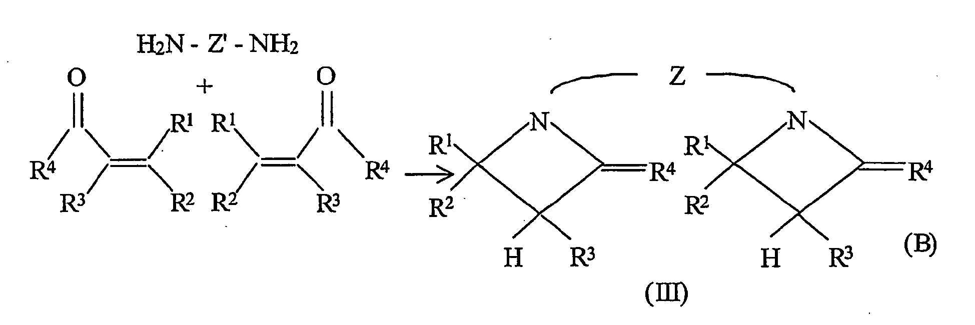

Es ist im Rahmen der vorliegenden Erfindung auch möglich, dass das Polyamin NH₂-Z'-NH₂ drei oder vier NH₂-Gruppen aufweist. In diesen Fällen werden durch Umsetzung von 1 Mol des Polyamins mit drei oder vier Mol des α, β-ungesättigten Ketons R¹R²C=CR³-COR⁴ die entsprechende Tris- oder Tetrakis-Azetidine erhalten.

Die Herstellung der erfindungsgemäßen Azetidin-Derivate ist relativ unproblematisch und kann durch Umsetzung des Polyamins mit dem α, β-ungesättigten Aldehyd oder Keton in Gegenwart eines organischen Lösemittels, insbesondere Toluol, im Temperaturbereich von 20 bis 150 °C, insbesondere unter Wasserabscheidung, erfolgen.

Die erfindungsgemäß bereitgestellten Azetidin-Derivate eignen sich hervorragend als latente Härterkomponente für Harze mit funktionellen Gruppen, die gegenüber Aminen reaktiv sind. Latente Härterkomponenten bewirken eine feuchtigkeitsinduzierte Härtung.

Vorzugsweise werden die erfindungsgemäßen Azetidin-Derivate für die Härtung von Polyurethan- und/oder Epoxidharzen eingesetzt.

Es ist im Rahmen der vorliegenden Erfindung jedoch ohne weiteres möglich, die Azetidin-Derivate bei anderen Polymersystemen wie z.B. Polyacrylaten oder anderen Polymerverbindungen zu verwenden, die mindestens eine gegenüber Aminen reaktive Gruppe aufweisen. Gemäß einer bevorzugten Ausführungsform wird hierbei das Azetidin-Derivat der Formel I über das sekundäre Amin an das zu härtende Harz addiert. Bei Feuchtigkeitseinwirkung wird dann der Azetidin-Ring hydrolytisch geöffnet und das dabei entstehende sekundäre Amin kann schließlich mit den reaktiven funktionellen Gruppen des zu härtenden Harzes abreagieren.

Diese Aushärtung des Gemisches bestehend aus Härterkomponente und Harz erfolgt vorzugsweise im Temperaturbereich von 5 bis 80 °C, insbesondere bei 10 bis 60 °C.

Die Menge der eingesetzten Härterkomponente ist relativ unkritisch, doch hat es sich aus wirtschaftlichen Gründen als besonders vorteilhaft erwiesen, das erfindungsgemäß vorgeschlagene Azetidin-Derivat in einer Menge von 0,01 bis 150 Gew.-%, insbesondere 0,1 bis 20 Gew.-%, bezogen auf die Menge des zu härtenden Harzes zu verwenden.

Aufgrund der besonderen Vorteile der erfindungsgemäßen Härterkomponente wie gute Herstellbarkeit, hohe Umweltfreundlichkeit (es werden keine flüchtigen Verbindungen während der Härtung abgespalten) sowie gute Lagerstabilität der Harz/Härter-Gemische eignen sich Azetidin-Derivate gemäß Formeln (II) und (III) hervorragend für einkomponentige, feuchtigkeitserhärtende Polymermassen, die vor allem für die . Herstellung von Dichtmassen, Klebstoffen und Beschichtungsmitteln, insbesondere von Bodenbeschichtungsmitteln, von besonderem Interesse sind.

Die nachfolgenden Beispiele sollen die Erfindung näher veranschaulichen.

### Beispiele

Alle Beispiele wurden unter Luftausschluss in Stickstoffatmosphäre hergestellt.

### Beispiel 1 A

### Herstellung eines Bis-azetidins auf Basis von Jeffamin D-230

In einem Reaktionsgefäß mit Wasserabscheider werden 30 g (0,306 mol) Mesityloxid, 33,48 g (0,146 mol) Jeffamin D-230 (Firma Huntsman), 0,1 g p-Toluolsulfonsäure in 150 g abs. Toluol gelöst und zum Sieden erhitzt. Die Reaktionsmischung wird solange bei Siedetemperatur gehalten, bis kein Wasser mehr über den Wasserabscheider entfernt werden kann (Theorie: 5,24 g Wasser). Anschließend wird das Toluol vollständig entfernt. Man erhält ein leicht orangefarbenes öliges Produkt.

### Beispiel 1 B

### Herstellung eines NCO-haltigen Prepolymers mit dem Bisazetidin-Härterderivat aus Bsp. 1A

In einem Reaktionsgefäß werden 250 g (0,125 mol) Polypropylenglycol Dow Voranol P2000 (von der Firma Dow) mit 55,55 g (0,25 mol) Isophorondiisocyanat und 0,1 g T12-DBTL bei 85°C gehalten, bis der theoretische NCO-Gehalt von 3,44 Gew.-% erreicht ist.
Anschließend werden in 300,0 g des NCO-haltigen Prepolymer bei Raumtemperatur 48,0 g (0,123 mol) Bisazetidin-Derivat aus Bsp. 1A eingerührt.
Das erhaltene Produkt ist harzig, durchsichtig klar und besitzt eine schwache orange Färbung. Auf einer Glasplatte aufgestrichen härtet es in kurzer Zeit ohne unangenehme Geruchsbelästigung vollständig durch. Auch in der Dickschichtanwendung erfolgt eine vollständige Durchhärtung.

### Beispiel 1 C

### Herstellung eines NCO-haltigen Prepolymers mit dem Bisazetidin-Härterderivat aus Bsp. 1A

In einem Reaktionsgefäß werden 250 g (0,125 mol) Polyproylenglycol Dow Voranol P2000 (von der Firma Dow) mit 43,54 g (0,25 mol) Toluoldiisocyanat und 0,1 g T12-DBTL bei 85°C gehalten, bis der theoretische NCO-Gehalt von 3,58 Gew.-% erreicht ist.
Anschließend werden in 250,0 g des NCO-haltigen Prepolymer bei Raumtemperatur 40,0 g (0,102 mol) Bisazetidin-Derivat aus Bsp. 1A eingerührt.
Das erhaltene Produkt ist harzig, durchsichtig klar und besitzt eine schwache orange Färbung. Auf einer Glasplatte aufgestrichen härtet es ohne unangenehme Geruchsbelästigung vollständig durch. Auch in der Dickschichtanwendung erfolgt eine vollständige Durchhärtung.

### Beispiel 2 A

### Herstellung eines Bis-azetidins auf Basis von 1,6-Hexamethylendiamin

In einem Reaktionsgefäß mit Wasserabscheider werden 30 g (0,306 mol) Mesityloxid, 16,91 g (0,146 mol) 1,6-Hexamethylendiamin, 0,1 g p-Toluolsulfonsäure in 150 g abs. Toluol gelöst und zum Sieden erhitzt. Die Reaktionsmischung wird solange bei Siedetemperatur gehalten, bis kein Wasser mehr über den Wasserabscheider entfernt werden kann (Theorie: 5,24 g Wasser). Anschließend wird das Toluol vollständig entfernt.

### Beispiel 2 B

### Herstellung eines NCO-haltigen Prepolymers mit dem Bisazetidin-Härterderivat aus Bsp. 2A

In einem Reaktionsgefäß werden 250 g (0,125 mol) Polyproylenglycol Dow Voranol P2000 (von der Firma Dow) mit 42,05 g (0,25 mol) 1,6-Hexamethylendüsocyanat und 0,1 g T12-DBTL bei 85°C gehalten, bis der theoretische NCO-Gehalt von 3,60 Gew.-% erreicht ist.
Anschließend werden in 250 g des NCO-haltigen Prepolymer bei Raumtemperatur 28,33 g (0,102 mol) Bisazetidin-Derivat aus Bsp. 2A eingerührt.
Das erhaltene Produkt ist harzig, durchsichtig klar und besitzt eine schwache gelbliche Färbung. Auf einer Glasplatte aufgestrichen härtet es ohne unangenehme Geruchsbelästigung vollständig durch. Auch in der Dickschichtanwendung erfolgt eine vollständige Durchhärtung.

### Beispiel 2 C

### Herstellung eines NCO-haltigen Prepolymers mit dem Bisazetidin-Härterderivat aus Bsp. 2A

In einem Reaktionsgefäß werden 250 g (0,125 mol) Polyproylenglycol Dow Voranol P2000 (von der Firma Dow) mit 65,59 g (0,25 mol) H12MDI und 0,1 g T12-DBTL bei 85°C gehalten, bis der theoretische NCO-Gehalt von 3,33 Gew.-% erreicht ist. Anschließend werden in das NCO-haltige Prepolymer bei Raumtemperatur 35,78 g (0,129 mol) Bisazetidin-Derivat aus Bsp. 2A eingerührt.
Das erhaltene Produkt ist harzig, durchsichtig klar und besitzt eine schwache gelbliche Färbung. Auf einer Glasplatte aufgestrichen härtet es ohne unangenehme Geruchsbelästigung vollständig durch. Auch in der Dickschichtanwendung erfolgt eine vollständige Durchhärtung.

### Beispiel 2 D

### Formulierung eines Epoxy-funktionellen Harzes mit dem Bisazetidin-Härterderivat aus Bsp. 2A

Bei 40°C werden in 250 g (0,735 mol) Bisphenol-A-diglycidylether 203,2 g (0,735 mol) Bisazetidin-Derivat aus Bsp. 2A homogen eingerührt.
Das erhaltene Produkt ist viskos, durchsichtig klar und besitzt eine schwache gelbliche Färbung. Auf einer Glasplatte aufgestrichen härtet es ohne unangenehme Geruchsbelästigung vollständig durch.

### Beispiel 3 A

### Herstellung eines Tris-azetidins auf Basis von Jeffamin T-403

In einem Reaktionsgefäß mit Wasserabscheider werden 30 g (0,306 mol) Mesityloxid, 45,85 g (0,102 mol) Jeffamin T-403 (Firma Huntsman), 0,1 g p-Toluolsulfonsäure in 150 g abs. Toluol gelöst und zum Sieden erhitzt. Die Reaktionsmischung wird solange bei Siedetemperatur gehalten, bis kein Wasser mehr über den Wasserabscheider entfernt werden kann (Theorie: 5,50 g Wasser). Anschließend wird das Toluol vollständig entfernt. Man erhält ein leicht orangefarbenes harziges Öl.

### Beispiel 3 B

### Herstellung eines NCO-haltigen Prepolymers mit dem Tris-azetidin-Härterderivat aus Bsp. 3A

In einem Reaktionsgefäß werden 250 g (0,125 mol) Polyproylenglycol Dow Voranol P2000 (von der Firma Dow) mit 42,05 g (0,25 mol) 1,6-Hexamethylendiisocyanat und 0,1 g T12-DBTL bei 85°C gehalten, bis der theoretische NCO-Gehalt von 3,60 Gew.-% erreicht ist.
Anschließend werden in das NCO-haltige Prepolymer bei Raumtemperatur 57,66 g (0,0835 mol) Tris-azetidin-Derivat aus Bsp. 3A eingerührt.
Das erhaltene Produkt ist harzig, durchsichtig klar und besitzt eine schwache orange Färbung. Auf einer Glasplatte aufgestrichen härtet es ohne unangenehme Geruchsbelästigung vollständig durch. Auch in der Dickschichtanwendung erfolgt eine vollständige Durchhärtung.

### Beispiel 3 C

### Formulierung eines Epoxy-funktionellen Harzes mit dem Tris-azetidin-Härterderivat aus Bsp. 3A

Bei 40°C werden in 250 g (0,735 mol) Bisphenol-A-diglycidylether 338,26 g (0,490 mol) tris-azetidin-Derivat aus Bsp. 3A homogen eingerührt.
Das erhaltene Produkt ist viskos, durchsichtig klar und besitzt eine schwache gelbliche Färbung. Auf einer Glasplatte aufgestrichen härtet es ohne unangenehme Geruchsbelästigung vollständig durch.

### Beispiel 4 A

### Herstellung eines Tris-azetidins auf Basis von Jeffamin T-403

In einem Reaktionsgefäß mit Wasserabscheider werden 30 g (0,357 mol) 3-Methylcrotonaldehyd, 53,50 g (0,119 mol) Jeffamin T-403 (Firma Huntsman), 0,1 g p-Toluolsulfonsäure in 200 g abs. Toluol gelöst und zum Sieden erhitzt. Die Reaktionsmischung wird solange bei Siedetemperatur gehalten, bis kein Wasser mehr über den Wasserabscheider entfernt werden kann (Theorie: 6,42 g Wasser). Anschließend wird das Toluol vollständig entfernt. Man erhält ein leicht orangefarbenes harziges Öl.

### Beispiel 4 B

### Herstellung eines NCO-haltigen Prepolymers mit dem Tris-azetidin-Härterderivat aus Bsp. 4A

In einem Reaktionsgefäß werden 250 g (0,125 mol) Polyproylenglycol Dow Voranol P3 000 (von der Firma Dow) mit 28,03 g (0,167 mol) 1,6-Hexamethylendiisocyanat und 0,1 g T12-DBTL bei 85°C gehalten, bis der theoretische NCO-Gehalt von 2,52 Gew.-% erreicht ist.
Anschließend werden in das NCO-haltige Prepolymer bei Raumtemperatur 36,03 g (0,0556 mol) Tris-azetidin-Derivat aus Bsp. 4A eingerührt.
Das erhaltene Produkt ist harzig, durchsichtig klar und besitzt eine schwache orange Färbung. Auf einer Glasplatte aufgestrichen härtet es ohne unangenehme Geruchsbelästigung vollständig durch. Auch in der Dickschichtanwendung erfolgt eine vollständige Durchhärtung.

### Beispiel 5 A

### Herstellung eines Bis-azetidins auf Basis von Jeffamin D-230

In einem Reaktionsgefäß mit Wasserabscheider werden 30 g (0,357 mol) 3-Methylcrotonaldehyd, 41,00 g (0,178 mol) Jeffamin D-230 (Firma Huntsman), 0,1 g p-Toluolsulfonsäure in 150 g abs. Toluol gelöst und zum Sieden erhitzt. Die Reaktionsmischung wird solange bei Siedetemperatur gehalten, bis kein Wasser mehr über den Wasserabscheider entfernt werden kann (Theorie: 6,42 g Wasser). Anschließend wird das Toluol vollständig entfernt. Man erhält ein leicht orangefarbenes Öl.

### Beispiel 5 B

### Formulierung eines Epoxy-funktionellen Harzes mit dem Bis-azetidin-Härterderivat aus Bsp. 5A

Bei 40°C werden in 250 g (0,735 mol) Bisphenol-A-diglycidylether 266,25 g (0,735 mol) Bis-azetidin-Derivat aus Bsp. 5A homogen eingerührt. Das erhaltene Produkt ist viskos, durchsichtig klar und besitzt eine schwache gelbliche Färbung. Auf einer Glasplatte aufgestrichen härtet es ohne unangenehme Geruchsbelästigung vollständig durch.

### Beispiel 6

### Lagerstabilitätsprüfung

Die gemäß den Beispielen 1 bis 5 hergestellten Gemische aus Prepolymeren und latenten Härtern werden einer Lagerung in geschlossenen Gefäßen bei Raumtemperatur (20-25°C) unterzogen und dabei folgende Ergebnisse erhalten:
Nach einer Lagerzeit von 12 Monaten bei einer Temperatur zwischen 20-25°C in licht- und luftdichten Gefäßen wurde bei allen Beispielen keine nennenswerte Farbveränderung festgestellt. Der Viskositätsanstieg über diesen Zeitraum war sehr gering (Anstieg um einen Faktor im Bereich von 1,1 - 1,3 gegenüber der Anfangsviskosität) und zeigte keinerlei Auswirkungen auf die Aushärtung bzw. die Verarbeitbarkeit.

## Patentansprüche

1. Azetidin-Derivate der allgemeinen Formeln (II) oder (III) 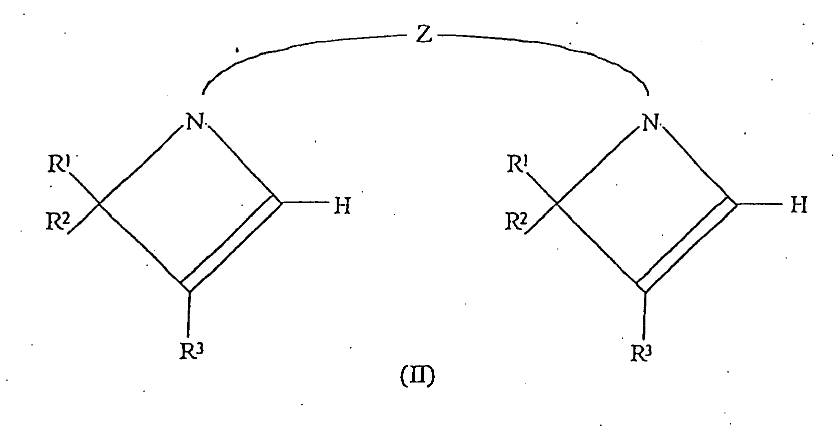 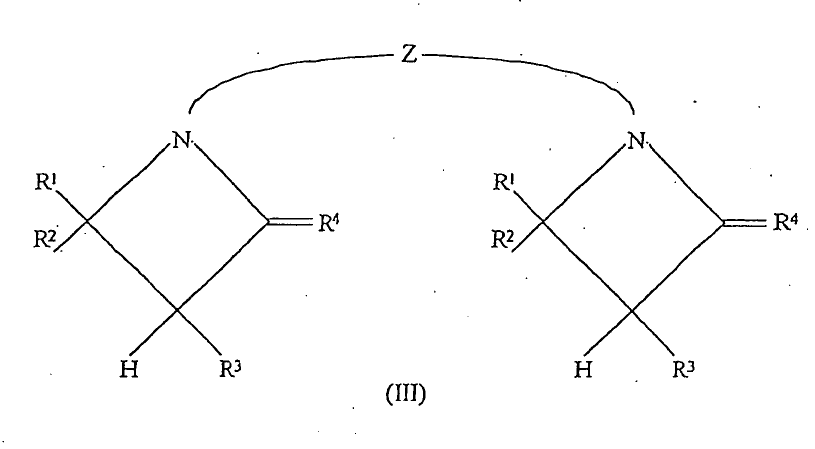 wobei
R¹, R² und R³ unabhängig voneinander H, C₁-C₂₀-Alkyl, C₃-C₈-Cycloalkyl, C₆-C₁₀-Aryl oder Alkylaryl mit C₁-C₄-Alkyl und C₆-C₁₀-Arylgruppen
R⁴ = C₁-C₆-Alkyl(iden)
Z = C₂-C₂₅-Alkylen, C₅-C₂₅-Cycloalkylen, C₆-C₂₄-Arylen sowie 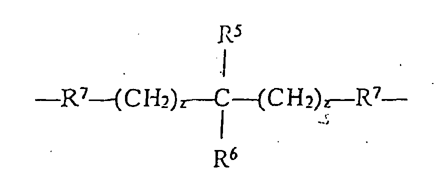
R⁵ und R⁶ = H, CH₂OH, C₁-C₄-AlkyJ, C₆H₅, 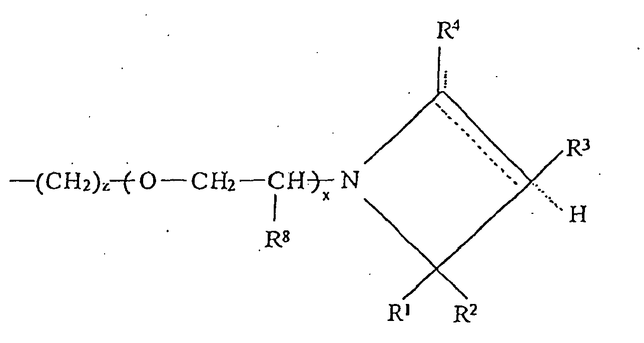
R⁷ = 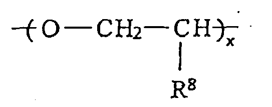
R⁸ = H, CH₃, C₂H₅, C₆H₅
z = 0 oder 1
x = 0 bis 100
bedeuten.

2. Verfahren zur Herstellung von Azetidin-Derivaten nach Anspruch 1, **dadurch gekennzeichnet, dass** man ein Polyamin der Formel NH₂-Z'-NH₂ mit einem α,β-ungesättigten Aldehyd der Formel R¹R²-C=CR³CHO oder einem α,β-ungesättigten Keton der Formel R¹R²C=CR³-COR⁴ im Temperaturbereich von 20 bis 150 °C umsetzt, wobei Z' für
C₂-C₂₅-Alkylen, C₅-C₂₅-Cycloalkylen, C₆-C₂₄-Arylen sowie 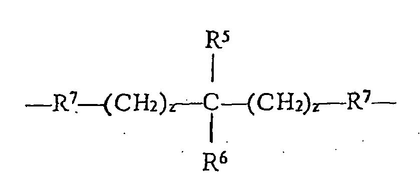
R⁵ und R⁶ = H, CH₂OH, C₁-C₄-Alkyl, C₆H₅, 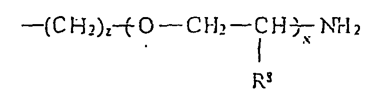
R7 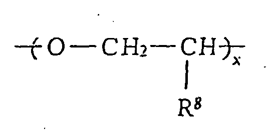
R⁸ = H, CH₃, C₂H₅, C₆H₅
z = 0 oder 1
x = 0 bis 100
stehen und R¹, R², R³, R⁴ vorstehende Bedeutung besitzen.

3. Verfahren nach Anspruch 2, **dadurch gekennzeichnet, dass** man die Umsetzung in Gegenwart eines organischen Lösemittels, insbesondere Toluol, durchführt.

4. Verwendung der Azetidin-Derivate nach Anspruch 1 als latente Härterkomponente für Harze mit gegenüber Aminogruppen reaktiven funktionellen Gruppen.

5. Verwendung nach Anspruch 4, **dadurch gekennzeichnet, dass** man das Azetidin-Derivat der Formel (II) und/oder (III) mit dem zu härtenden Harz vermischt, den Azitidin-Ring durch Feuchtigkeitseinwirkung hydrolytisch öffnet und das dabei entstehende sekundäre Amin mit den reaktiven funktionellen Gruppen des zu härtenden Harzes abreagieren lässt.

6. Verwendung nach einem der Ansprüche 4 oder 5, **dadurch gekennzeichnet, dass** man als zu härtendes Harz Polyurethane oder Polyepoxide sowie Mischungen davon einsetzt.

7. Verwendung nach einem der Ansprüche 4 bis 6, **dadurch gekennzeichnet, dass** man die Härterkomponente in einer Menge von 0,01 bis 150 Gew.-%, insbesondere 0,1 bis 20 Gew.-%, bezogen auf die Menge des zu härtenden Harzes verwendet.

8. Verwendung nach einem der Ansprüche 4 bis 7, **dadurch gekennzeichnet, dass** man die Aushärtung des Gemisches, bestehend aus Härterkomponente und Harz, bei einer Temperatur von 5 bis 80 °C und ggf. in Gegenwart eines geeigneten Katalysators durchführt.

9. Verwendung nach einem der Ansprüche 4 bis 8, **dadurch gekennzeichnet, dass** die Härterkomponente bei der Herstellung von (Boden-)Beschichtungen, Dicht- und Klebstoffen eingesetzt wird.

## Claims

1. Azetidine derivatives of the general formula (II) or (III) 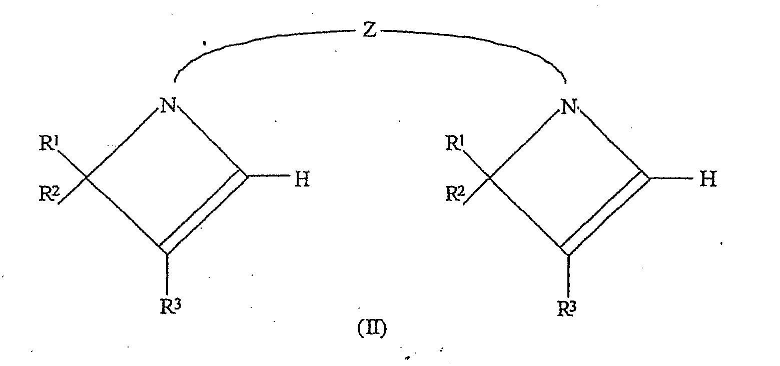 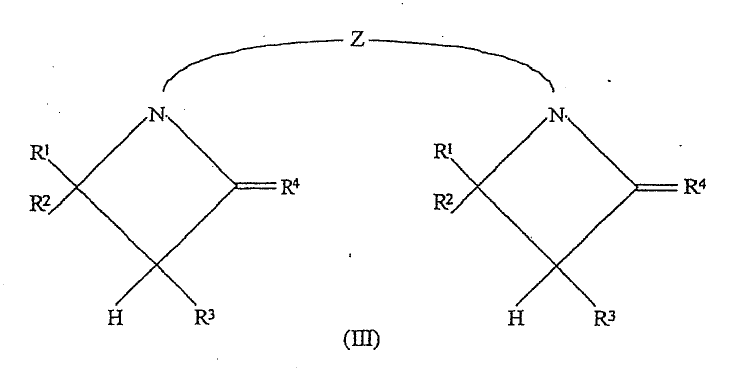 where
R¹, R² and R³ independently of one another are H, C₁-C₂₀ alkyl, C₃-C₈ cycloalkyl, C₆-C₁₀ aryl or alkylaryl with C₁-C₄ alkyl and C₆-C₁₀ aryl groups
R⁴ = C₁-C₆ alkyl(idene)
Z = C₂-C₂₅ alkylene, C₅-C₂₅ cycloalkylene, C₆-C₂₄ arylene and also 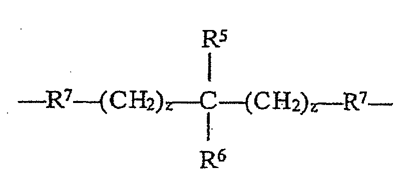
R⁵ and R⁶ H, CH₂OH, C₁-C₄ alkyl, C₆H₅, 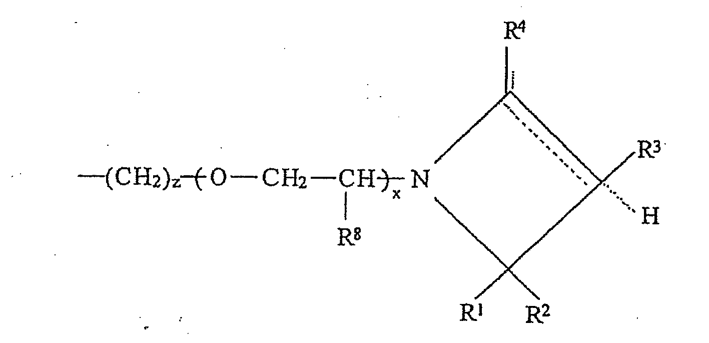
R⁷ 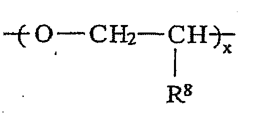
R⁸ = H, CH₃, C₂H₅, C₆H₅
z = 0 or 1
x = 0 to 100.

2. Method for producing azetidine derivatives according to Claim 1, **characterized in that** a polyamine of the formula NH₂-Z'-NH₂ is reacted with an α,β-unsaturated aldehyde of the formula R¹R²-C=CR³CHO or with an α,β-unsaturated ketone of the formula R¹R²C=CR³-COR⁴ in the temperature range from 20 to 150°C, where Z' is
C₂-C₂₅ alkylene, C₅-C₂₅ cycloalkylene, C₆-C₂₄ arylene and also 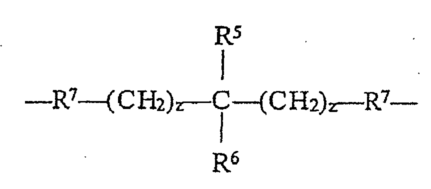
R⁵ and R⁶ = H, CH₂OH, C₁-C₄ alkyl, C₆H₅, 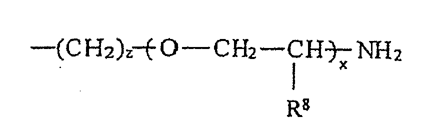
R⁷ = 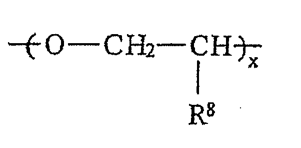
R⁸ = H, CH₃, C₂H₅, C₆H₅
z = 0 or 1
x = 0 to 100
and R¹, R², R³, and R⁴ possess the above definition.

3. Method according to Claim 2, **characterized in that** the reaction is carried out in the presence of an organic solvent, especially toluene.

4. Use of the azetidine derivatives according to Claim 1 as a latent curing component for resins having functional groups which are reactive toward amino groups.

5. Use according to Claim 4, **characterized in that** the azetidine derivative of the formula (II) and/or (III) is mixed with the resin to be cured, the azetidine ring is hydrolytically opened by moisture exposure, and the secondary amine formed is caused to react with the reactive functional groups of the resin to be cured.

6. Use according to either of Claims 4 and 5, **characterized in that** polyurethanes or polyepoxides and also mixtures thereof are used as resin to be cured.

7. Use according to any one of Claims 4 to 6, **characterized in that** the curing component is used in an amount of 0.01% to 150% by weight, in particular 0.1% to 20% by weight, based on the amount of the resin to be cured.

8. Use according to any one of Claims 4 to 7, **characterized in that** the mixture consisting of curing component and resin is cured at a temperature of 5 to 80°C and optionally in the presence of a suitable catalyst.

9. Use according to any one of Claims 4 to 8, **characterized in that** the curing component is used in the production of (floor) coatings, sealants, and adhesives.

## Revendications

1. Dérivés d'azétidine de formules générales (II) ou (III) 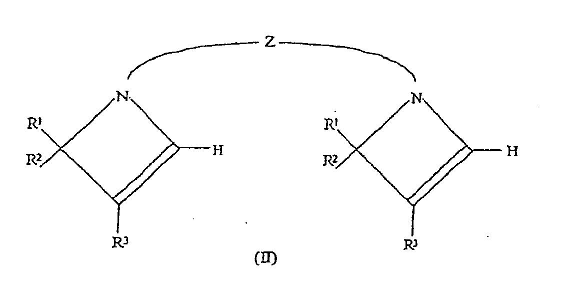 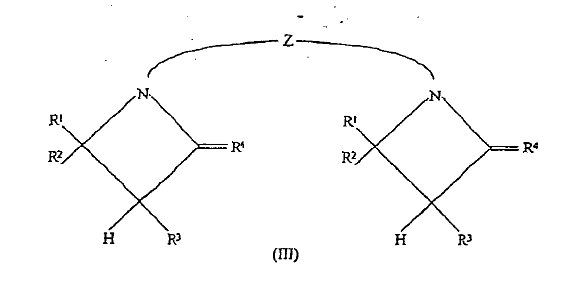 dans lesquelles
R¹, R² et R³ représentent, chacun indépendamment, H, un groupe alkyle en C₁-C₂₀, cycloalkyle en C₃-C₈, aryle en C₆-C₁₀ ou alkylaryle à fragments alkyle en C₁-C₄ et aryle en C₆-C₁₀,
R⁴ représente un groupe alkyl(idèn)e en C₁-C₆,
Z représente un groupe alkylène en C₂-C₂₅, cycloalkylène en C₅-C₂₅, arylène en C₆-C₂₄ ainsi que 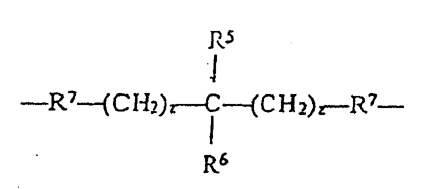
R⁵ et R⁶ = H, CH₂OH, alkyle en C₁-C₄, C₆H₅, 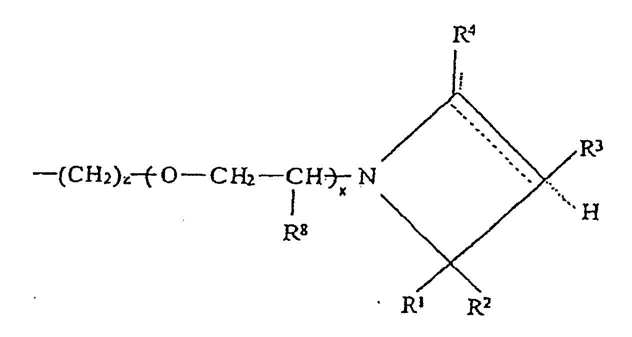
R⁷ 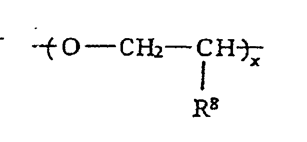
R⁸ = H, CH₃, C₂H₅, C₆H₅,
z = 0 ou 1,
x = 0 à 100.

2. Procédé pour la préparation de dérivés d'azétidine selon la revendication 1, **caractérisé en ce qu'**on fait réagir une polyamine de formule NH₂-Z'-NH₂ avec un aldéhyde α,β-insaturé de formule R¹R²-C=CR³CHO ou une cétone α,β-insaturée de formule R¹R²C=CR³-COR⁴ dans la plage de température de 20 à 150 °C, Z' représentant un groupe alkylène en C₂-C₂₅, cycloalkylène en C₅-C₂₅, arylène en C₆-C₂₄ ainsi que 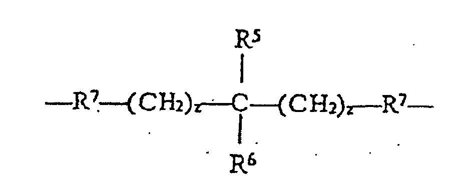
R⁵ et R⁶ = H, CH₂OH, alkyle en C₁-C₄, C₆H₅, 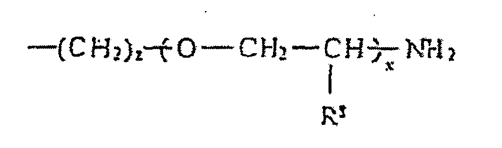
R⁷ = 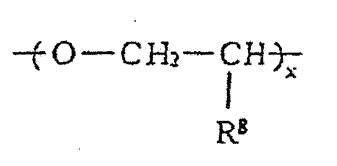
R⁸ = H, CH₃, C₂H₅, C₆H₅,
z = 0 ou 1,
x = 0 à 100
et R¹, R², R³, R⁴ ont les significations précédentes.

3. Procédé selon la revendication 2, **caractérisé en ce que** la réaction s'effectue en présence d'un solvant organique, en particulier le toluène.

4. Utilisation des dérivés d'azétidine selon la revendication 1, en tant que composant durcisseur latent pour des résines à groupes fonctionnels réactifs vis-à-vis de groupes amino.

5. Utilisation selon la revendication 4, **caractérisée en ce qu'**on mélange le dérivé d'azétidine de formule (II) et/ou de formule (III) avec la résine à durcir, on ouvre le cycle azétidine par hydrolyse sous l'effet de l'humidité et on laisse réagir complètement l'amine secondaire ainsi formée avec les groupes fonctionnels réactifs de la résine à durcir.

6. Utilisation selon la revendication 4 ou 5, **caractérisée en ce qu'**on utilise comme résine à durcir des polyuréthannes ou des polyépoxydes ainsi que des mélanges de ceux-ci.

7. Utilisation selon l'une quelconque des revendications 4 à 6, **caractérisée en ce qu'**on utilise le composant durcisseur en une quantité de 0,01 à 150 % en poids, en particulier de 0,1 à 20 % en poids, par rapport à la quantité de la résine à durcir.

8. Utilisation selon l'une quelconque des revendications 4 à 7, **caractérisée en ce qu'**on effectue le durcissement du mélange, consistant en composant durcisseur et résine, à une température de 5 à 80 °C et éventuellement en présence d'un catalyseur approprié.

9. Utilisation selon l'une quelconque des revendications 4 à 8, **caractérisée en ce qu'**on utilise le composant durcisseur dans la production de revêtements (de sol), de matériaux d'étanchéité et d'adhésifs.
